(19) Europäisches Patentamt
European Patent Office
Office européen des brevets

(11) EP 0 621 892 B1

(12) # FASCICULE DE BREVET EUROPEEN

(45) Date de publication et mention de la délivrance du brevet:
**08.07.1998 Bulletin 1998/28**

(21) Numéro de dépôt: **94900130.9**

(22) Date de dépôt: **11.11.1993**

(51) Int Cl.$^6$: **C11B 9/00**, C07D 307/83

(86) Numéro de dépôt international:
**PCT/EP93/03164**

(87) Numéro de publication internationale:
**WO 94/12143 (09.06.1994 Gazette 1994/13)**

(54) **UTILISATION DE FURANONES A TITRE D'INGREDIENTS PARFUMANTS**

Verwendung von Furanonen wie Duftstoffe

USE OF FURANONES AS SCENTING INGREDIENTS

(84) Etats contractants désignés:
**CH DE FR GB LI NL**

(30) Priorité: **18.11.1992 CH 3533/92**

(43) Date de publication de la demande:
**02.11.1994 Bulletin 1994/44**

(73) Titulaire: **FIRMENICH SA**
**1211 Genève 8 (CH)**

(72) Inventeur: **GAUDIN, Jean-Marc**
**F-74100 Annemasse (FR)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:

**EP-A- 0 041 122** **EP-A- 0 167 265**
**EP-A- 0 219 199** **US-A- 5 114 493**
**US-A- 5 137 035**

- **DATABASE WPI Section Ch, Week 8213, Derwent Publications Ltd., London, GB; Class D18, AN 82-24625E & JP,A,57 029 279 (JAPAN TOBACCO & SALT) 17 Février 1982**
- **AGRICULTURAL AND BIOLOGICAL CHEMISTRY vol. 37, no. 10 , 1973 , TOKYO JP pages 2441 - 2442 I.SAKATA ET AL. 'Isolation and identification of 2,3 dimethyl-4-hydroxy-2-nonenoic acid lactone from shubi'**
- **FOOD TECHNOLOGY vol. 44, no. 2 , Février 1990 , CHICAGO US G.A.BURDOCK ET AL. '15.GRAS substances'**
- **JOURNAL OF THE AMERICAN CHEMICAL SOCIETY vol. 111 , 1989 , WASHINGTON, DC US pages 5472 - 5474 E.J.COREY ET AL 'Enantiospecific total synthesis of pseudopterosins A and E' cité dans la demande**
- **TETRAHEDRON LETTERS vol. 32, no. 38 , 1991 , OXFORD GB pages 5191 - 5192 M.CARDA ET AL. 'Total synthesis of (-).mintlactone' cité dans la demande**
- **CHEMICAL AND PHARMACEUTICAL BULLETIN vol. 31, no. 8 , 1983 , TOKYO JP pages 2639 - 2651 I.KITAGAWA ET AL. 'Chemical transformation of terpenoids.V.Acidic conversions of 10-hydroxygeraniol and 10-hydroxynerol derivatives leading to cyclic monoterpenoids' cité dans la demande**
- **JOURNAL OF ORGANIC CHEMISTRY vol. 47 , 1982 , EASTON US pages 741 - 743 Y.ITO ET AL. 'A new approach for stereoselective synthesis of gamma-butyrolactones' cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 621 892 B1

Printed by Jouve, 75001 PARIS (FR)

## Description

### Domaine technique

La présente invention a trait au domaine de la parfumerie. Plus particulièrement, elle concerne l'utilisation d'une furanone de formule

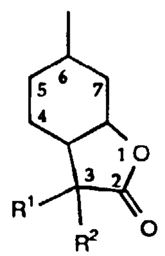

(I)

dans laquelle les symboles $R^1$ et $R^2$, pris séparément, sont identiques et représentent chacun un radical méthyle, ou sont différents et représentent chacun un atome d'hydrogène ou un radical méthyle, ou, pris ensemble, représentent un radical méthylène.

### Technique antérieure

Les furanones de formule (I) sont des homologues de la 5,6,7,7a-tétrahydro-7a-méthoxy-3,6-diméthyl-2(4H)-benzo[b]furanone, aussi connue sous le nom de menthalactone, un composant naturel de l'huile essentielle de la menthe poivrée et dont l'utilisation en parfumerie et dans l'industrie des arômes est décrite dans l'art antérieur [voir le brevet US 4,407,740].

D'autre part, le brevet US 5,114,493 décrit l'utilisation pour l'aromatisation du tabac de lactones de formule

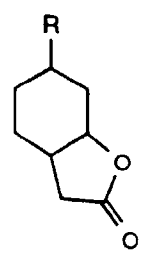

dans laquelle R est un radical alkyle de $C_1$ à $C_6$, un radical $C_1$-$C_4$ alkylphényle ou un radical $C_1$-$C_4$ alkoxyphényle.

Les structures des furanones (I) sont également connues de l'art antérieur. Par exemple, la synthèse de la perhydro-6-méthyl-3-méthylène-2-benzo[b]furanone a fait l'objet de plusieurs publications [voir, par exemple, J. M. Fang et al., Proc. Nat. Sci. Counc. ROC(A) 9, 95 (1985)], tandis que la perhydro-3,6-diméthyl-2-benzo[b]furanone a été décrite dans le brevet EP 219 199, ayant trait à un procédé pour la préparation de lactones. Bien que ce brevet ne contienne aucune référence à des propriétés particulières de cette furanone, on connait toutefois l'utilité de ce composé à titre de produit insectifuge - voir la demande de brevet européen EP 167 265 -.

La synthèse de la perhydro-3,3,6-triméthyl-2-benzo[b]furanone a aussi été décrite par I. Yoshihiko et al. dans le J. Org. Chem. 47, 741 (1982).

D'autre part, plusieurs isomères optiquement actifs des composés (I), dont les structures comportent plusieurs centres chiraux, ont été décrits dans la littérature, leur caractérisation étant toutefois souvent déficiente [voir, par exemple, J. M. Fang et al., réf. citée ; M. Carda et al., Tetrahedron Lett. **32**, 5191 (1991); I. Kitagawa et al., Chem. Pharm. Bull. **31**, 2639 (1983) ; E. J. Corey et al., J. Am. Chem. Soc. **111**, 5472 (1989); C. W. Jefford et al., J. Chem. Soc. Chem. Comm. **1988**, 634].

Malgré cette abondance de descriptions de nature synthétique et structurelle et la similarité chimique entre les furanones(I) et leurs homologues connus de US 4,407,740 et 5,114,493, à notre connaissance les propriétés odorantes de ces furanones (I) sont passées jusqu'ici complètement inaperçues. Nous n'avons trouvé dans l'art antérieur cité aucune référence, ni même suggestion, de l'éventuelle utilité de ces furanones, ou de l'un de leurs isomères, à titre

d'ingrédient parfumant.

**Exposé de l'invention**

Or, nous avons maintenant découvert que ces composés possèdent des propriétés olfactives tout à fait surprenantes au vu de l'art antérieur et qui rendent leur utilisation en parfumerie très intéressante.

Par exemple, la perhydro-6-méthyl-3-méthylène-2-benzo[b]furanone possède une odeur très puissante de type coumariné, gras, lactonique, avec une note de fond balsamique rappelant la jonquille. Il s'agit d'une note odorante très proche de celle de la coumarine et qui se trouve représentée à son mieux chez la (+)-(3aS,6R,7aR)-perhydro-6-méthyl-3-méthylène-2-benzo[b]furanone, citée ici à titre préférentiel.

Pour sa part, la perhydro-3,6-diméthyl-2-benzo[b]furanone possède elle une note odorante de type coumariné très puissante, avec un côté foin rappelant l'odeur de la flouve et du Florex® [5 et 6-éthylidèneoctahydro-5,8-méthano-2H-1-benzopyran-2-one ; origine: Firmenich SA, Genève, Suisse], ainsi qu'une sous-note fruitée-balsamique évoquant l'odeur du tonka et de la réglisse.

Nous avons découvert avec surprise que l'odeur de ce composé se révèle particulièrement intéressante, car, de l'avis des parfumeurs, il s'agit de la note la plus proche olfactivement de celle de la coumarine que l'on connait actuellement. Ceci ressort clairement des essais de comparaison, présentés plus loin, entre cette furanone et les composés actuellement disponibles sur le marché dont les odeurs sont apparentées à celle de la coumarine.

Les propriétés odorantes des furanones (I) apparaissent comme tout à fait surprenantes au vu de l'art antérieur. En effet, leur odeur est complètement différente, en qualité et intensité, de celle de leur homologue connu mentionné plus haut, la menthalactone. Comme il ressort des exemples de comparaison présentés plus loin, cette dernière possède une note odorante dont la puissance est de plusieurs ordres de grandeur inférieure à celle des furanones (I) mentionnées plus haut. Sa note est moins coumarinée et plus lactonique que celle de ces dernières et ne possède pas le caractère de type foin qui est typique de la coumarine et que l'on retrouve également dans l'odeur des furanones (I), particulièrement chez la perhydro-3,6-diméthyl-2-benzo[b]furanone. En plus, cette dernière possède également une note fraîche-aromatique, rappelant la lévo-carvone, note qui est totalement absente chez la menthalactone, qui exhale elle une odeur beaucoup plus fruitée.

D'autre part, la menthalactone n'est pas du tout substantive, contrairement aux furanones (I). Ceci ressort clairement du test comparatif de substantivité présenté plus loin.

Nous avons également découvert, de façon inattendue, que les nombreux isomères optiquement actifs de la perhydro-3,6-diméthyl-2-benzo[b]furanone possèdent tous, de façon plus ou moins marquée, la note coumarinique recherchée, accompagnée de nuances olfactives variées qui rendent tous ces composés utiles pour la préparation de compositions et articles parfumés.

Parmi ces isomères, on citera à titre préférentiel les (+)-(3R,3aS,6R,7aR)-perhydro-3,6-diméthyl-2-benzo[b]furanone et (+)-(3S,3aS,6R,7aR)-perhydro-3,6-diméthyl-2-benzo[b]furanone. Ces deux composés possèdent des notes odorantes coumarinées puissantes, l'odeur du premier étant moins lactonique mais plus douce que celle du deuxième, avec un caractère de type caramel. La note du second composé cité, qui possède au mieux les caractères de type tonka, fèves, foin, flouve mentionnés plus haut, est aussi plus coumarinée et plus puissante que celle du premier, dont le caractère de type coumarine est plus marqué en fond qu'en tête et accompagné d'un côté métallique. Des mélanges parfumants contenant des quantités prépondérantes de l'un ou l'autre de ces composés, ont également été jugés des ingrédients parfumants fort utiles selon l'invention.

Par ailleurs, nous avons été surpris de constater que des mélanges de ces isomères optiquement actifs pouvaient parfois avoir des performances olfactives égales, ou même supérieures à celles de leurs ingrédients individuels et se révéler des ingrédients parfumants de choix. Nous avons notamment observé un effet d'exhaltation des qualités odorantes des deux composés préférés cités ci-dessus lorsqu'on mélangeait ces composés entre eux, ou qu'on employait leur mélange avec d'autres isomères dans des compositions contenant des quantités prépondérantes dudit mélange des (+)-(3R,3aS,6R,7aR)-perhydro-3,6-diméthyl-2-benzo[b]furanone et (+)-(3S,3aS,6R,7aR)-perhydro-3,6-diméthyl-2-benzo[b]furanone. Ces compositions, contenant 50% ou plus de ce mélange, possédaient les notes odorantes caractéristiques de la perhydro-3,6-diméthyl-2-benzo[b]furanone citées plus haut, mais leur odeur apparaissait comme plus intense et plus riche dans le caractère coumarinique et lactonique recherché que celles des isomères individuels composant le mélange. Il s'agit de compositions nouvelles, possédant des propriétés inattendues par rapport à l'art antérieur, et qui constituent ainsi l'un des objets de l'invention.

D'autre part, lorsque de tels mélanges résultent directement de certains procédés de synthèse, ils présentent en plus un avantage économique par rapport aux formes optiquement actives pures dont la synthèse est plus onéreuse.

Parmi les isomères de la perhydro-3,6-diméthyl-2-benzo[b]furanone, on peut encore citer à titre préférentiel la (-)-(3S,3aR,6R,7aS)-perhydro-3,6-diméthyl-2-benzo[b]furanone qui possède une odeur coumarinée, flouve, foin, vaguement soufrée.

Lorsqu'on fait référence à la perhydro-3,6-diméthyl-2-benzo[b] furanone dans les exemples d'application, on entend se référer aussi bien au mélange racémique qu'à l'un des isomères optiquement actifs, notamment à ceux qui sont des composés préférés, ou encore à des compositions contenant au moins 50% en poids de l'un des isomères (+)-(3R,3aS,6R,7aR) et (+)-(3S,3aS,6R,7aR), ou de leur mélange.

De par leurs qualités olfactives, les furanones (I), leurs isomères optiquement actifs, ainsi que les mélanges de ces isomères, sont des ingrédients parfumants qui se prêtent tout aussi bien pour des applications en parfumerie fine qu'en parfumerie fonctionnelle. Ils conviennent pour la préparation de bases et compositions parfumantes, parfums et eaux de toilette, ainsi que pour le parfumage d'articles divers tels des savons, des gels de bain ou douche, des shampoings et autres produits d'hygiène capillaire, des désodorisants corporels ou d'air ambiant et des préparations cosmétiques. Leur note odorante étant d'une excellente tenacité, on peut les utiliser avantageusement pour le parfumage de détergents et d'adoucissants textiles. Ils trouvent également un emploi heureux dans des produits d'entretien.

Dans ces applications, ils seront utilisés soit seuls, soit, comme il est plus courant dans l'art, en mélange avec d'autres ingrédients parfumants, des solvants ou des adjuvants usuels en parfumerie.

Les concentrations dans lesquelles ils peuvent être utilisés dépendent de l'effet olfactif recherché, ainsi que de la nature des coingrédients avec lesquels ils sont mélangés dans les compositions parfumantes et articles parfumés les contenant. Ces concentrations peuvent donc varier dans une gamme de valeurs très large. A titre d'exemple, on peut citer des concentrations de l'ordre de 1 à 10%, voire même 20% ou plus, en poids, par rapport au poids de la composition dans laquelle ils sont incorporés, ceci lors de leur usage dans la préparation de bases et compositions parfumantes. Des valeurs bien inférieures à celles-ci seront généralement utilisées lors de l'emploi de ces composés dans le parfumage des divers articles de consommation cités précédemment.

**Manières de réaliser l'invention**

Les composés (I) ont été préparés à partir de produits commerciaux ou qui peuvent être facilement obtenus de façon connue en soi. Les schémas suivants illustrent les procédés utilisés pour chaque cas particulier.

## SCHEMA I

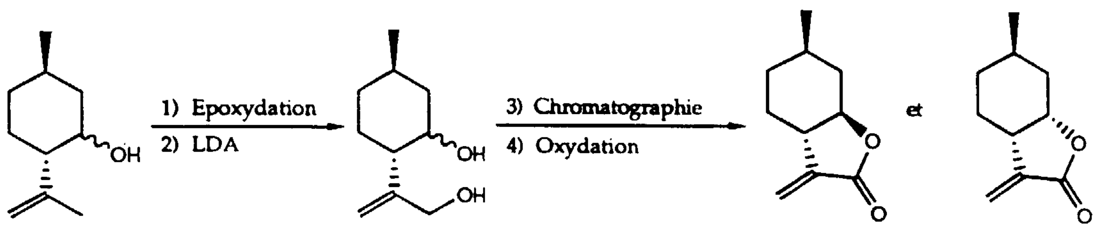


et

(+)-(3aS,6R,7aR) (3aS,6R,7aS)

Le produit de départ (Fluka) dans ce schéma est un mélange de (-)-isopulégol (72%) et de (+)-néoisopulégol (20%). L'époxydation de ce mélange par l'acide metachloroperbenzoïque dans le dichlorométhane [voir T.-L. Ho et al., Synth. Comm. 19, 813 (1989) et T. G. Waddell et al., J. Org. Chem. 52, 4802 (1987) pour des réactions analogues] et traitement avec 2,5 éq. de lithium diisopropylamine (LDA) dans le THF [voir, par exemple, K.-H. Schulte-Elte, Helv. Chim. Acta 50, 153 (1967) pour des réactions analogues] fournissent un mélange des diols indiqués, lesquels peuvent ensuite être séparés par chromatographie préparative et oxydés à l'aide d'oxyde d'argent pour fournir les deux lactones indiquées à l'état pur.

D'autres méthodes de préparation de ces lactones sont décrites, par exemple, par S.B. Balkrishna et al., Heterocycles 16, 2091 (1981) et T. J. Brocksom et al., Synth. Comm. 18, 1403 (1988).

Lorsqu'on part du (-)-isopulégol pur (Fluka) et qu'on soumet le diol intermédiaire à une hydrogénation catalytique, comme illustré au schéma suivant:

## SCHEMA II

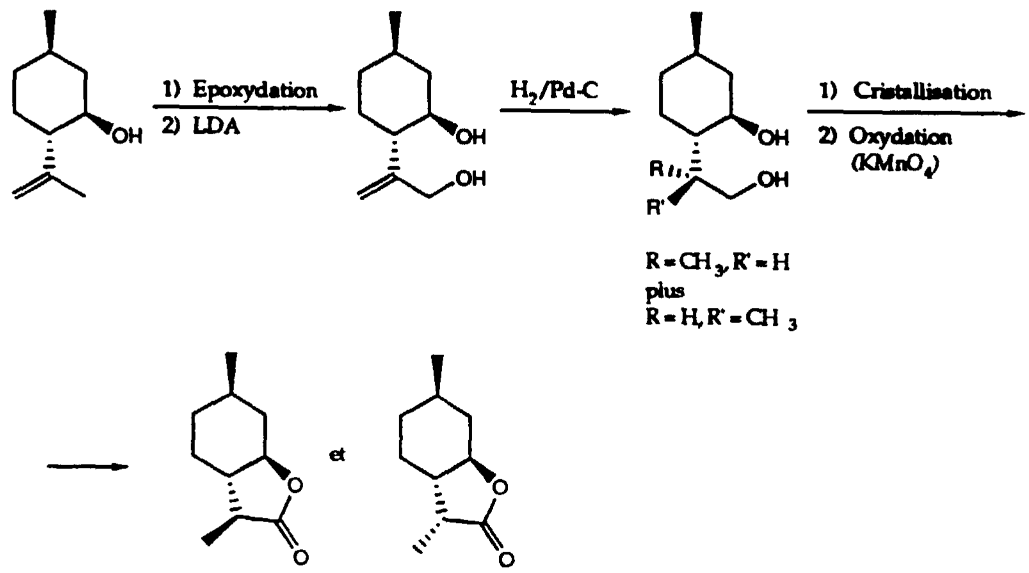


(+)-(3S,3aS,6R,7aR) (+)-(3R,3aS,6R,7aR)

on obtient le mélange des diols indiqués. Ces diols sont séparés par cristallisation dans l'héxane et ensuite oxydés à l'aide de permanganate de potassium [voir C. W. Jefford et al., J. Chem. Soc., Chem. Comm. 1988, 634] pour fournir les deux lactones indiquées à l'état pur.

Le schéma III ci-après illustre la préparation de deux autres isomères de la perhydro-3,6-diméthyl-2-benzo[b] furanone, à partir du (+)-néoisopulégol pur (obtenu par séparation chromatographique à partir du mélange technique cité au schéma I), par hydroboration de ce dernier dans le THF [procédé analogue à celui décrit par K.-H. Schulte-Elte, référence citée], suivie d'oxydation du mélange de diols obtenu.

## SCHEMA III

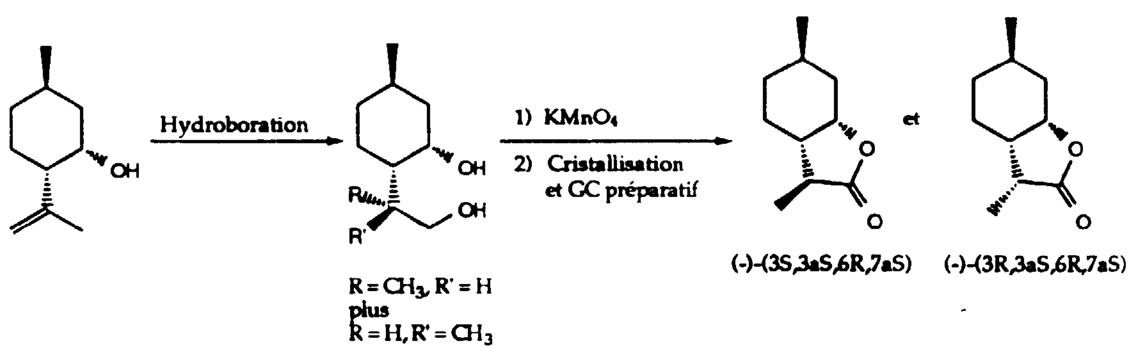


(-)-(3S,3aS,6R,7aS) (-)-(3R,3aS,6R,7aS)

On obtient un mélange des deux lactones désirées, dont on sépare l'isomère majoritaire par cristallisation dans l'hexane, et l'isomère minoritaire par chromatographie préparative en phase gazeuse des eaux mères.

Comme il est illustré au schéma IV ci-après

## SCHEMA IV

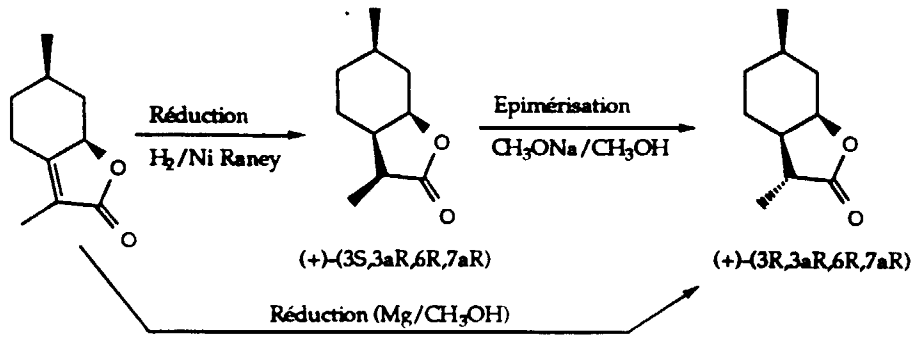

(+)-(3S,3aR,6R,7aR)
(+)-(3R,3aR,6R,7aR)

la réduction catalytique de la (-)-menthalactone donne la lactone de configuration (3S,3aR,6R,7aR), alors que la réduction du même produit de départ par le magnésium dans le méthanol [voir T. Hudlicky et al., Tetrahedron Lett. 28, 5287 (1987) et H. Yoda et al., Chem. Lett. 1989, 1391, pour des réactions analogues] produit la lactone de configuration (3R,3aR,6R,7aR). Cette dernière peut également être préparée par épimérisation à l'aide de méthylate de sodium de son épinière indiqué.

La réduction de la (+)-(3S,3aR,6R,7aR)-perhydro-3,6-diméthyl-2-benzo[b]furanone préparée ci-dessus à l'aide de $LiAlH_4$ dans le diéthyléther a fourni le diol indiqué au schéma V ci-après:

## SCHEMA V

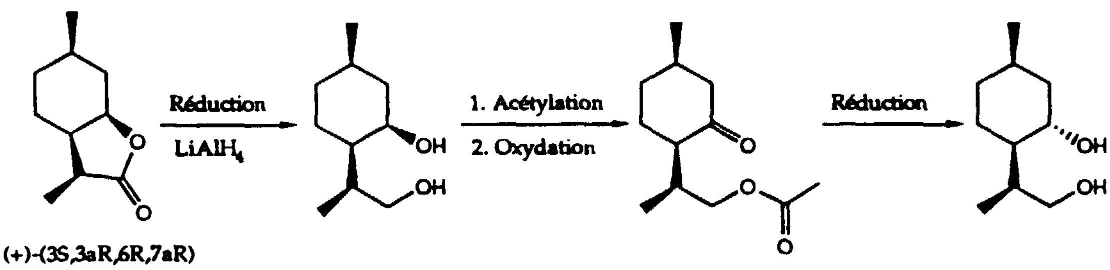

(+)-(3S,3aR,6R,7aR)

L'acétylation de ce diol avec un équivalent d'anhydride acétique dans la pyridine, suivie d'oxydation à l'aide du réactif de Jones a fourni la cétone intermédiaire, dont la réduction a permis d'obtenir le diol indiqué. Ce diol possédait la configuration appropriée pour fournir, par oxydation au permanganate de potassium, la lactone de configuration (3S, 3aR,6R,7aS), comme indiqué au schéma VI:

## SCHEMA VI

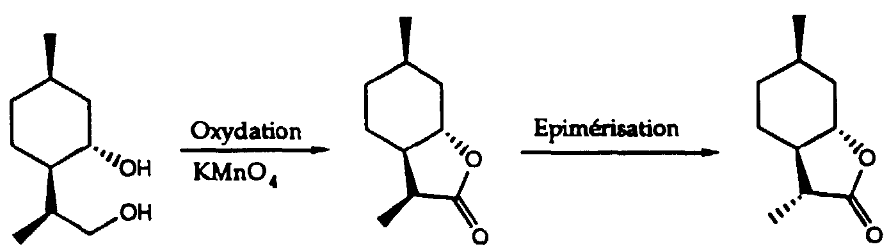

(-)-(3S,3aR,6R,7aS)
(-)-(3R,3aR,6R,7aS)

L'épimérisation de cette lactone dans des conditions basiques ($CH_3ONa$/THF) a fourni son épimère indiqué.

Toutes les lactones (I) dont la préparation est décrite ci-dessus possèdent un groupe méthyle de configuration R à la position 6. L'utilisation de produits de départ appropriés, ayant le méthyle concerné avec une configuration énantiomère de celle indiquée dans les schémas, a permis d'obtenir, bien entendu, les lactones (I) de configuration 6S correspondantes.

Les composés (I) ainsi obtenus ont été exhaustivement caractérisés à l'aide de techniques spectroscopiques (SM, RMN-COSY, nOe, etc ...) et leurs données analytiques sont présentées plus loin.

Lorsqu'on a utilisé, en tant que produits de départ, des mélanges d'isomères optiquement actifs, on a obtenu des mélanges correspondants de lactones (I), qui se sont révélés d'excellents ingrédients parfumants. Par exemple, lorsqu'on a appliqué au mélange technique de (-)-isopulégol et (+)-néoisopulégol cité au schéma I, la séquence de réactions décrite au schéma II, à l'exclusion de la séparation des diols par cristallisation, on a obtenu un mélange de lactones contenant environ 50% en poids de (+)-(3S,3aS,6R,7aR)-perhydro-3,6-diméthyl-2-benzo[b]furanone, 30% en poids de (+)-(3R,3aS,6R,7aR)-perhydro-3,6-diméthyl-2-benzo[b]furanone, 11% en poids de (-)-(3S,3aS,6R,7aS)-perhydro-3,6-diméthyl-2-benzo[b]furanone et 7% en poids de (-)-(3R,3aS,6R,7aS)-perhydro-3,6-diméthyl-2-benzo[b] furanone. Ce mélange possédait des propriétés olfactives qui le rendaient un ingrédient parfumant préféré pour les applications selon l'invention. Par ailleurs, ce mélange s'est révélé également utile en tant que produit de départ pour la synthèse de la (-)-(3aS,6R,7aS)-perhydro-3,3,6-triméthyl-2-benzo[b]furanone, décrite plus loin.

D'autres mélanges ont été obtenus à partir du même isopulégol de qualité technique de départ, mais en variant légèrement les conditions de synthèse. Par exemple, l'hydroboration de ce produit, suivie de l'oxydation avec $KMnO_4$, de façon analogue à celle illustrée au schéma m, a fourni un mélange contenant environ 13% en poids de (+)-(3S, 3aS,6R,7aR)-perhydro-3,6-diméthyl-2-benzo[b]furanone, 61% en poids de (+)-(3R,3aS,6R,7aR)-perhydro-3,6-diméthyl-2-benzo[b]furanone, 20% en poids de (-)-(3S,3aS,6R,7aS)-perhydro-3,6-diméthyl-2-benzo[b]furanone et 6% en poids de (-)-(3R,3aS,6R,7aS)-perhydro-3,6-diméthyl-2-benzo[b]furanone qui s'est aussi révélé un ingrédient parfumant intéressant.

Ces mélanges possédant une quantité prépondérante des deux isomères préférés de la perhydro-3,6-diméthyl-2-benzo[b]furanone, à savoir le (+)-(3S,3aS,6R,7aR) et le (+)-(3R,3aS,6R,7aR), développaient en effet des odeurs coumarinées très puissantes, avec les caractères foin, flouve typiques déjà mentionnés auparavant.

Pour ce qui est des produits (I) racémiques, ils peuvent être préparés selon les méthodes décrites dans l'art antérieur cité plus haut, ou bien, selon les méthodes décrites, par exemple, aux schémas I et II, mais en partant de produits de départ racémiques.

Les méthodes de préparation des composés (I) sont décrites en détail ci-après.

A. Préparation de la (+)-(3S,3aS,6R,7aR)-perhydro-3,6-diméthyl-2-benzo[b]furanone et de la (+)-(3R,3aS,6R,7aR)-perhydro-3,6-diméthyl-2-benzo[b]furanone

A 40,7 g (264 mmole) de (-)-(1R,3R,4S)-8-p-menthén-3-ol ($[\alpha]_D^{20}$ = -7,74°) dans le dichlorométhane (130 ml) on a ajouté 64,5 g (317 mmole) d'acide m-chloroperbenzoïque. Le mélange a été agité pendant 4 h à température ambiante, ensuite filtré, lavé avec $NaHCO_3$, de l'eau et de la saumure. On a séché la phase organique, concentré et distillé le produit brut. Une solution de 16,15 g (95 mmole) de produit ainsi distillé dans le THF (30 ml) a été ajoutée lentement, à 0°C, à 295 mmole de LDA dans le THF (170 ml). Le mélange a été chauffé à 45°C pendant 1 h et ensuite refroidi à température ambiante. Le mélange réactionnel a été versé sur glace et extrait à l'éther. La phase organique a été lavée à la saumure, séchée et concentrée pour fournir 14 g de produit brut. Ce dernier a été recristallisé dans l'hexane pour fournir 11,96 g de (1R,3R,4S)-8(10)-p-menthéne-3,9-diol à l'état pur.
15 G (88 mmole) de ce diol ont été hydrogénés avec 0,15 g de Pd/C à 10%, dans l'acétate d'éthyle (150 ml), à une pression atmosphérique de $H_2$. Le produit brut (15,7 g) a été distillé pour fournir 10,6 g (rend. 70%) d'un mélange de (1R,3R,4S,8S)-3,9-p-menthanediol et (1R,3R,4S,8R)-3,9-p-menthanediol. Ces deux diols ont ensuite été séparés par cristallisation dans l'hexane.

(1R,3R,4S,8S)-3,9-p-menthanediol

| | |
|---|---|
| RMN($^1$H, 360MHz, $CDCl_3$) : | 0,85(d, J=7Hz, 3H); 0,89(m, 1H); 0,92(d, J=6Hz, 3H); 0,98(m, 2H); 1,35(m, 1H); 1,41 (m, 1H); 1,63(m, 2H); 1,99(dxm, J=13Hz, 1H); 2,07(m, 1H); 3,42(dxdxd, $J_1$=11, $J_2$=4, $J_3$=4Hz, 1H); 3,48(dxd, $J_1$=11, $J_2$=7Hz, 1H); 3,56(dxd, $J_1$=11, $J_2$=5Hz, 1H) δ ppm. |
| RMN($^{13}$C, 360MHz, $CDCl_3$) : | 12,6(q); 22,2(q); 25,3(t); 31,6(d); 34,4(t); 35,6(d); 45,2(t); 45,6(d); 66,6(t); 71,7(d) δ ppm. |
| SM : | 172(1, $M^+$), 154(3), 139(6), 123(16), 112(16), 95(38), 81(100), 71(63), 55(57), 41(32). |

(-)-(1R,3R,4S,8R)-3,9-p-menthanediol

$[\alpha]_D^{20}$ = -22,5°; c = 1,2%, dans le $CHCl_3$
P.f. 100-101°C

| | |
|---|---|
| RMN($^1$H, 360MHz, $CDCl_3$) : | 0,93(d, J=7Hz, 3H); 0,97(d, J=7Hz, 3H); 0,8-1,0(m, 2H); 1,24(dxdxdxd, $J_1$=11, $J_2$=11, $J_3$=11, $J_4$=3Hz, 1H); 1,35(dxm, J=11Hz, 1H); 1,42(m, 1H); 1,56(dxm, J=13Hz, 1H); 1,64(dxm, J=13Hz, 1H); 1,84(m, 1H); 1,97(dxm, J=13Hz, 1H); 3,44(dxdxd, $J_1$=10, $J_2$=10, $J_3$=4Hz, 1H); 3,58(dxd, $J_1$=11, $J_2$=3Hz, 1H); 3,65(dxd, $J_1$ =11, $J_2$=5Hz, 1H) δ ppm. |
| RMN($^{13}$C, 360MHz, $CDCl_3$) : | 12,0(q); 22,1(q); 29,5(t); 31,5(d); 34,7(t); 38,6(d); 44,6(t); 48,6(d); 67,0(t) ; 70,1(d) δ ppm. |
| SM : | 172(1, $M^+$), 154(3), 139(7), 124(18), 112(28), 95(33), 81(100), 71(62), 55(65), 41(35). |

Ces deux diols ont ensuite été oxydés séparemment, à l'aide de permanganate de potassium, de façon analogue à celle décrite sous B ci-après, pour fournir les lactones désirées à l'état pur.

(+)-(3R,3aS,6R,7aR)-perhydro-3,6-diméthyl-2-benzo[b]furanone (pure à 94%)

$[\alpha]_D^{20}$ = +85,0°; c = 0,7%, dans le $CHCl_3$
IR : 2940, 2883, 1808, 1158, 962 $cm^{-1}$

| | |
|---|---|
| RMN($^1$H, 360MHz, $CDCl_3$) : | 1,02(d, J=7Hz, 3H); 1,11(m, 1H); 1,15(d, J=7Hz, 3H); 1,24(dxdxd, $J_1$=11, $J_2$=11, $J_3$=11Hz, 1H); 1,34(m, 1H); 1,60(m, 1H); 1,77(m, 1H); 1,82(m, 1H); 1,93(m, 1H); 2,25 (dxdxd, $J_1$=11, $J_2$=4, $J_3$=4Hz, 1H); 2,64(dxq, $J_1$=7, $J_2$=7Hz, 1H); 4,00(dxdxd, $J_1$=11, $J_2$=11, $J_3$=4Hz, 1H) δ ppm. |
| RMN($^{13}$C, 360MHz, $CDCl_3$) : | 9,6(q); 22,0(q); 23,8(t); 31,3(d); 34,2(t); 38,8(t); 38,8(d); 47,2(d); 81,5(d); 180,4(s) δ ppm. |
| SM : | 167(1), 139(1), 124(4), 109(18), 95(27), 81(100), 67(73), 55(24), 41(30). Odeur: décrite dans l'introduction. |

(+)-(3S,3aS,6R,7aR)-perhydro-3,6-diméthyl-2-benzo[b]furanone (pure à 97%)

$[\alpha]_D^{20}$ = +19,5°; c = 1,25%, dans le $CHCl_3$
IR : 2939, 2884, 1809, 1175, 1093, 1006 $cm^{-1}$

| | |
|---|---|
| RMN($^1$H, 360MHz, $CDCl_3$) : | 1,02(d, J=7Hz, 3H) ; 1,03(m, 1H); 1,22(d, J=7Hz, 3H); 1,22(m, 1H); 1,26(m, 1H); 1,47 (dxdxdxd, $J_1$=13, $J_2$=13, $J_3$=13, $J_4$=4Hz, 1H); 1,63(m, 1H); 1,82(dxm, $J_1$=13Hz, 1H); 1,94(dxdxd, $J_1$=13, $J_2$=5, $J_3$=3Hz, 1H); 2,22(m, 1H); 2,24(m, 1H); 3,76(dxdxd, $J_1$=13, $J_2$=13, $J_3$=4Hz, 1H) δ ppm. |
| RMN($^{13}$C, 360MHz, $CDCl_3$) : | 12,6(q); 22,0(q); 26,7(t); 31,4(d); 34,2(t); 38,2(t); 41,4(d); 51,5(d); 82,5(d); 179,5(s) δ ppm. |
| SM : | 167(1), 139(1), 124(6), 109(16), 95(27), 81(100), 67(62), 55(18), 41(17). Odeur: décrite dans l'introduction. |

B. Préparation de la (-)-(3S,3aS,6R,7aS)-perhydro-3,6-diméthyl-2-benzo[b)furanone et de la (-)-(3R,3aS,6R,7aS)-perhydro-3,6-diméthyl-2-benzo[b]furanone

A une solution refroidie de (+)-(1R,3S,4S)-8-p-menthén-3-ol ($[\alpha]_D^{20}$ = +9,75°; 30,8 g; 200 mmole) et de borohydrure de sodium (4,18 g; 110 mmole) dans le THF (80 ml), maintenue sous azote, on a ajouté goutte à goutte, pendant 1 h, à une température comprise entre -10° et 0°C, 21,3 g (150 mmole) d'éthyl éthérate de trifluorure de bore. Une fois l'addition complétée, on a agité le mélange pendant 3 h à 0°C. 30 Ml d'eau ont alors été ajoutés lentement (15 min) et on a agité pendant encore 15 min. Toujours à 0°C, on a ajouté 80 g d'une solution à 12% de KOH dans l'éthanol, suivie, 20 min plus tard, de 22 g (460 mmole) de peroxyde d'hydrogène (solution à 70% dans l'eau). Une fois l'addition terminée, le mélange réactionnel a été agité à 20°C pendant encore 2 h. On a enlevé le THF, extrait le produit brut à l'éther et lavé à la saumure. Le mélange organique a été séché et concentré pour fournir 32 g d'une matière brute huileuse constituée par un mélange des diols (1R,3S,4S,8S)-3,9-p-menthanediol et (1R,3S,4S,8R)-3,9-p-menthanediol. Ce mélange de diols a ensuite été oxydé comme suit: 6,88 g (40 mmole) de cette huile, en solution dans 20

ml d'acétate d'éthyle, ont été ajoutés goutte à goutte pendant 10 min à 15,15 g (96 mmole) de permanganate de potassium dans 50 ml d'acétate d'éthyle. Le mélange a été agité pendant la nuit à température ambiante et ensuite hydrolysé par addition d'une solution saturée de bisulfite de sodium jusqu'à décoloration complète. Le précipité blanc a été filtré et la phase organique lavée à la saumure pour apporter son pH à 7, séchée et concentrée pour fournir 6,34 g d'un mélange des lactones désirées. La (-)-(3R,3aS,6R,7aS)-perhydro-3,6-diméthyl-2-benzo[b]furanone a été obtenue à partir de ce mélange par recristallisation dans l'hexane, alors que la (-)-(3S,3aS,6R,7aS)-perhydro3,6-diméthyl-2-benzo[b]furanone a été isolée des liqueurs mères par chromatographie préparative en phase gazeuse.

(-)-(3S,3aS,6R,7aS)-perhydro-3,6-diméthyl-2-benzo[b]furanone (pure à 98%)

$[\alpha]_D^{20}$ = -67,9°; c = 1,07%, dans le $CHCl_3$
P. f. 52-53°C
IR : 2937, 1804, 1161, 969 $cm^{-1}$

RMN($^1H$, 360MHz, $CDCl_3$) : 0,93(d, J=7Hz, 3H); 0,94(m, 1H); 1,25(m, 2H); 1,27(d, J=7Hz, 3H); 1,80(m, 3H); 1,95 (m, 1H); 2,17(dxm, $J_1$=14Hz, 1H); 2,36(q, J=7Hz, 1H); 4,69(dxd, $J_1$=4, $J_2$=4Hz, 1H) δ ppm.
RMN($^{13}C$, 360MHz, $CDCl_3$) : 14,1(q); 21,5(q); 26,0(d); 27,3(t); 31,6(t); 36,0(t); 41,3(d); 44,1(d); 77,4(d); 180,5(s) δ ppm.
SM : 167(1), 139(1), 124(13), 109(19), 95(100), 81(55), 67(72), 55(30), 41(38). Odeur: coumarinée, lactonique, côté tabac froid.

(-)-(3R,3aS,6R,7aS)-perhydro-3,6-diméthyl-2-benzo[b]furanone (pure à 88%)

$[\alpha]_D^{20}$ = -31,3°; c = 0,95%, dans le $CHCl_3$
P.f. 47-48°C
IR : 2938, 1806, 1158, 962 $cm^{-1}$

RMN($^1H$, 360MHz, $CDCl_3$) : 0,90(m, 1H); 0,92(d, J=7Hz, 3H); 1,11(m, 1H); 1,16(d, J=7Hz, 3H); 1,21(m, 1H); 1,57 (m, 1H); 1,67(m, 2H); 2,22(m, 2H); 2,79(dxq, $J_1$=7, $J_2$=7Hz, 1H); 4,44(m, 1H) δ ppm.
RMN($^{13}C$, 360MHz, $CDCl_3$) : 9,1(q); 22,0(q); 23,2(t); 26,2(d); 32,1(t); 36,2(t); 39,1(t); 42,3(d); 78,2(d); 179,8(s) δ ppm.
SM : 167(1), 139(1), 124(12), 109(18), 95(100), 81(38), 67(70), 55(27), 41(35). Odeur: coumarinée, grasse.

C Préparation de la (+)-(3R,3aR,6R,7aR)-perhydro-3,6-diméthyl-2-benzo[b]furanone

On a ajouté 1,70 g (70 mmole) de magnésium, par portions, à une solution de la (-)-(6R,7aR)-5,6,7,7a-tétrahydro-3,6-diméthyl-2(4H)-benzo[b]furanone (0,83 g; 5 mmole) dans le THF (50 ml). La réaction exothermique a été maintenue sous agitation à une température proche de la température ambiante à l'aide d'un bain d'eau, pendant la nuit. Le mélange de la réaction a été hydrolysé avec une solution à 10% de HCl et extrait à l'éther. La phase organique a été lavée à la saumure, séchée et concentrée. La purification par "flash chromatography" a fourni 743 mg (rend. 88%) de la furanone en titre, pure à 99%.
$[\alpha]_D^{20}$ = +45,7°; c = 0,9%, dans le $CHCl_3$
IR : 2939, 1802, 1169, 1007 $cm^{-1}$

RMN($^1H$, 360MHz, $CDCl_3$) : 0,96(d, J=7Hz, 3H); 0,98(m, 2H) ; 1,21(d, J=7Hz, 3H) ; 1,40(m, 1H); 1,56(dxm, $J_1$=13Hz, 1H); 1,67(m, 1H); 1,86(dxm, $J_1$=l4Hz, 1H); 2,08(m, 1H); 2,22(m, 1H); 2,48 (dxq, $J_1$=14, $J_2$=7Hz, 1H); 4,49(dxdxd, $J_1$ =12, $J_2$=7, $J_3$=7Hz, 1H) δ ppm.
RMN($^{13}C$, 360MHz, $CDCl_3$) : 13,3(q); 22,0(q); 24,0(t); 28,7(t); 29,5(d); 35,3(d); 37,8(t); 41,7(d); 77,5(d); 179,6(s) δ ppm.
SM : 169(1), 139(1), 124(3), 109(19), 95(44), 81(100), 67(65), 55(27), 41(32).

D. Préparation de la (+)-(35,3aR,6R,7aR)-perhydro-3.6-diméthyl-2-benzo[b]furanone

On a hydrogéné 10 g (60 mmole) de (-)-(6R,7aR)-5,6,7a-tétrahydro-3,6-diméthyl-2(4H)-benzo[b]furanone dans le méthanol (40 ml) avec du Ni de Raney (3 g), sous 50 atmosphères de $H_2$, pendant 3 jours, à température ambiante. L'extraction à l'éther du produit de la réaction a fourni 9,7 g (rend. 96%) de la furanone désirée, que l'on a purifiée pour

obtenir un produit pur à 98%.

$[\alpha]_D^{20}$ = +9,2°; c = 1,1%, dans le $CHCl_3$

IR : 2943, 1803, 1156, 972 $cm^{-1}$

RMN($^1H$, 360MHz, $CDCl_3$) : 1,02(d, J=7Hz, 3H) ; 1,18(d, J=7Hz, 3H) ; 1,33(m, 1H); 1,50(m, 3H); 1,89(m, 3H); 2,32 (m, 1H); 2,77(dxq, $J_1$=7, $J_2$=7Hz, 1H); 4,52(dxd, $J_1$=5, $J_2$=5Hz, 1H) δ ppm.

RMN($^{13}C$, 360MHz, $CDCl_3$) : 9,7(q); 17,4(t); 19,8(q); 25,3(d); 28,9(t); 33,1(t); 38,8(d); 41,3(d); 77,9(d); 179,7(s) δ ppm.

SM : 167(1), 139(1), 124(10), 109(21), 95(100), 81(55), 67(76), 55(35), 41(44). Odeur: lactonique.

E. Préparation de la (-)-(3S,3aR,6R,7aS)-perhydro-3,6-diméthyl-2-benzo[b]furanone

On a ajouté lentement 9,7 g (57,7 mmole) de (+)-(3S,3aR,6R,7aR)-perhydro-3,6-diméthyl-2-benzo[b]furanone (préparée sous D) à 2,2 g (57 mmole) de $LiAlH_4$ dans l'éther (200 ml). La réaction exothermique a été maintenue à environ 0°C à l'aide d'un bain de glace. Une fois la réaction terminée, on a dilué à l'éther et hydrolysé le mélange avec une faible quantité de saumure. Le solide granulaire blanc a été enlevé par filtration et lavé à l'éther. La phase organique a été filtrée et évaporée pour fournir 7,5 g (rend. 76%) de (1R,3R,4R,8S)-3,9-p-menthanediol, dont les données analytiques étaient les suivantes:

RMN($^1H$, 360MHz, $CDCl_3$) : 0,89(m, 2H); 0,95(d, J=7Hz, 3H); 1,15(d, J=7Hz, 3H); 1,30(dxdxd, $J_1$=13, J2=9, $J_3$=4Hz, 1H); 1,44(m, 1H); 1,53(m, 1H); 1,68(m, 2H); 1,85(m, 2H); 3,40(dxd, $J_1$=8, $J_2$=4Hz, 1H); 3,50(dxd, $J_1$=11, $J_2$=8Hz, 1H); 3,94(m, 1H) δ ppm.

RMN($^{13}C$, 360MHz, $CDCl_3$) : 17,3(t); 17,6(q); 21,4(q); 27,4(d); 32,1(t); 38,5(d); 39,1(t); 46,4(d); 64,7(t); 71,3(d) δ ppm.

SM : 172(1, $M^+$), 154(4), 139(8), 123(28), 112(32), 95(69), 81(100), 71(80), 55(73), 41(44).

On a ajouté, à -5°C, à une solution du diol préparé ci-dessus (8 g; 46,7 mmole) dans la pyridine (50 ml) 4,8 g d'anhydride acétique. Le mélange de la réaction a ensuite été agité pendant la nuit à température ambiante. Après extraction à l'éther, la phase éthérée a été lavée successivement avec une solution à 10% de HCl, de l'eau et de la saumure. On a séché sur sulfate de magnésium et concentré. Le mélange a été soumis à une "flash chromatography" (éluant : pentane/éther = 5/1) pour fournir 7,8 g (rend. 78%) d'acétate de (1R,3R,4R,8S)-3-hydroxy-9-p-menthanyle dont les données analytiques étaient les suivantes:

RMN($^1H$, 360MHz, $CDCl_3$) : 1,27(d, J=7Hz, 3H); 1,30(d, J=7Hz, 3H); 1,40(m, 3H); 1,50(m, 1H); 1,55-1,75(m, 3H); 1,82(m, 1H); 1,91(m, 1H); 2,07(s, 3H); 3,94(dxd, $J_1$=11, $J_2$=7Hz, 1H); 4,03(m, 1H); 4,20(dxd, $J_1$=11, $J_2$=4Hz, 1H) δ ppm.

RMN($^{13}C$, 360MHz, $CDCl_3$) : 16,5(q); 21,0(q); 21,2(t); 21,4(q); 28,0(d); 31,1(t); 33,0(d); 39,2(t); 43,3(d); 68,3(t); 70,2 (d); 171,5(s) δ ppm.

SM : 171(1), 154(9), 136(15), 121(13), 112(58), 97(52), 81(57), 69(58), 55(53), 43(100).

Du réactif de Jones a été ajouté goutte à goutte à une solution de 7,44 g (34,8 mmole) de l'acétate susmentionné dans l'acétone (100 ml) jusqu'à ce que l'oxydation, suivie par chromatographie en couche mince, soit complète. On a filtré le mélange réactionnel sur Celite® et évaporé, extrait à l'éther et lavé la solution éthérée trois fois à l'eau. L'évaporation de l'éther a fourni 7,29 g (rend. 99%) d'acétate de (1R,4R,8S)-3-oxo-9-p-menthanyle ayant les données suivantes:

RMN($^1H$, 360MHz, benzene) : 0,70(d, J=7Hz, 3H); 0,87(d, J=7Hz, 3H); 1,15(m, 1H); 1,31(m, 1H); 1,43(m, 1H); 1,54 (m, 1H); 1,72(m, 1H); 1,72(s, 3H); 1,87(dxd, $J_1$=13, $J_2$=8Hz, 1H); 2,03(m, 2H); 2,11 (dxd, $J_1$=13, $J_2$=5Hz, 1H); 3,93(dxd, $J_1$=10, $J_2$=5Hz, 1H); 4,02(dxd, $J_1$=10, $J_2$=4Hz, 1H) δ ppm.

RMN($^{13}C$, 360MHz, $CDCl_3$) : 15,5(q); 20,6(q); 20,9(q); 26,3(t); 29,9(t); 31,6(d); 33,4(d); 48,3(t); 52,7(d); 66,8(t); 171,1(s); 212,6(s) δ ppm.

SM : 212(1, $M^+$), 169(1), 152(6), 137(11), 123(8), 112(100), 97(17), 69(29), 43(33).

On a ajouté en petites portions, à -60°C et pendant 2 h, 2,9 g (415 mmole) de lithium à une solution contenant 5,78 g (27,3 mmole) de l'acétate préparé ci-dessus, 33 g (620 mmole) de chlorure d'ammonium dans 300 ml de $NH_3$ liquide et 100 ml d'éther. Le $NH_3$ liquide a été évaporé et 100 ml d'éther et 100 ml d'eau ajoutés. On a extrait la phase

organique avec de la saumure pour obtenir 4,7 g de produit brut. La purification par "flash chromatography" de ce dernier (éluant: pentane/éther = 1/2) a fourni le (+)(1R,3S,4R,8S)-3,9-p-menthanediol, avec les données suivantes : $[\alpha]_D^{20}$ = +8,7° ; c = 1,9%, dans le $CHCl_3$

| | |
|---|---|
| RMN($^1H$, 360MHz, $CDCl_3$) : | 0,95(d, J=7Hz, 3H); 0,98(d, J=7Hz, 3H); 1,33-1,51(m, 6H); 1,78(m, 2H); 2,07(m, 1H); 3,57(dxd, $J_1$=11, $J_2$=4Hz, 1H); 3,65(dxd, $J_1$=11, $J_2$=5Hz, 1H); 3,70(dxdxd, $J_1$=9, $J_2$=4, $J_3$=4Hz, 1H) δ ppm. |
| RMN($^{13}C$, 360MHz, $CDCl_3$) : | 12,4(q); 18,7(q); 24,3(t); 28,1(d); 31,1(t); 38,4(d); 41,0(t); 48,8(d); 66,3(d); 66,9(t) δ ppm. |
| SM : | 154(6), 137(11), 123(25), 95(55), 81(100), 67(60), 55(65), 41(45). |

3,09 G (18 mmole) de ce diol, en solution dans 8 ml d'acétate d'éthyle, ont été ajoutés à 6,92 g (44 mmole) de permanganate de potassium dans 25 ml d'acétate d'éthyle. Le mélange de la réaction a été agité à température ambiante pendant la nuit et ensuite hydrolysé par addition de solution de bisulfite de sodium jusqu'à décoloration complète. On a filtré le précipité blanc et lavé la phase organique avec de la saumure jusqu'à pH 7. Après séchage et concentration on a obtenu 1,97 g de (-)(3S,3aR,6R,7aS)-perhydro-3,6-diméthyl-2-benzo[b]furanone pure à 95%. Les données de cette furanone sont présentées ci-après :
$[\alpha]_D^{20}$ = -71,7° ; c = 0,8%, dans le $CHCl_3$
IR : 2943, 1807, 1186, 989 $cm^{-1}$

| | |
|---|---|
| RMN($^1H$, 360MHz, $CDCl_3$) : | 1,07(d, J=7Hz, 3H); 1,17(d, J=7Hz, 3H); 1,4-1,7(m, 4H); 1,72(dxd, $J_1$=12, $J_2$=5Hz, 1H); 1,94(m, 1H); 2,10(dxdxd, $J_1$=12, $J_2$=3, $J_3$=3Hz, 1H); 2,30(m, 1H); 2,65(dxq, $J_1$=7, $J_2$=7Hz, 1H); 4,23(dxdxd, $J_1$=12, $J_2$=12, $J_3$=4Hz, 1H) δ ppm. |
| RMN($^{13}C$, 360MHz, $CDCl_3$) : | 9,6(q); 19,2(q); 20,2(t); 28,1(d); 31,0(t); 36,1(t); 38,9(d); 48,1(d); 78,2(d); 180,3(s) δ ppm. |
| SM : | 167(1), 124(3), 109(15), 95(22), 81(100), 67(57), 55(20), 41(15). |
| Odeur : | décrite dans l'introduction. |

F. Préparation de la (-)-(3R,3aR,6R,7aS)-perhydro-3,6-diméthyl-2-benzo[b]furanone

Une solution de 0,33 g (2 mmole) de la furanone obtenue sous E et 0,064 g (1,2 mmole) de méthylate de sodium anhydre dans le THF (6 ml) a été agitée pendant 4 h à température ambiante. La réaction a été arrêtée avec une solution à 10% de HCl et le mélange réactionnel extrait à l'éther. On a lavé la phase organique à la saumure, séché et concentré pour obtenir un mélange de lactones. La furanone désirée a été séparée de ce mélange par chromatographie préparative en phase gazeuse.
$[\alpha]_D^{20}$ = -9,8° ; c = 0,92%, dans le $CHCl_3$
IR : 2940, 1809, 1171, 999 $cm^{-1}$

| | |
|---|---|
| RMN($^1H$, 360MHz, $CDCl_3$) : | 1,07(d, J=7Hz, 3H); 1,23(d, J=7Hz, 3H); 1,48(m, 2H); 1,62(m, 2H); 1,70(dxd, $J_1$=12, $J_2$=5Hz, 1H); 1,80(m, 1H); 2,16(dxdxd, $J_1$=12, $J_2$=3, $J_3$=3Hz, 1H); 2,30(m, 2H); 3,99 (dxdxd, $J_1$=11, $J_2$=9, $J_3$=4Hz, 1H); δ ppm. |
| RMN($^{13}C$, 360MHz, $CDCl_3$) : | 12,5(q); 19,2(q); 23,0(t); 28,2(d); 31,0(t); 35,7(t); 41,5(d); 52,5(d); 79,3(d); 179,5(s) δ ppm. |
| SM : | 147(1), 140(2), 133(1), 124(3), 109(12), 95(22), 81(100), 67(52), 55(23), 41(18). |
| Odeur : | coumarinée, flouve, foin, soufrée, caoutchouc. |

G. Préparation de (+)-(3aS,6R,7aR)-perhydro-6-méthyl-3-méthylène-2-benzo[b]furanone et de (3aS,6R,7aS)-perhydro-6-méthyl-3-méthylène-2-benzo[b]furanone

Ces deux lactones, préparées selon le schéma I, ou comme il est décrit dans l'art antérieur cité auparavant, avaient les données suivantes:

(+)-(3aS,6R,7aR)-perhydro-6-méthyl-3-méthylène-2-benzo[b]furanone $[\alpha]_D^{20}$ = +55,22°

| | |
|---|---|
| RMN($^1H$, 360MHz, $CDCl_3$) : | 1,04(d, J=6Hz, 3H); 1,05-1,20(m, 1H); 1,25-1,45(m, 2H); 1,64(m, 1H); 1A4(dxm, J=14Hz, 1H); 2,13(dxm, J=14Hz, 1H); 2,26(dxdxd, $J_1$=13, $J_2$=2, $J_3$=2Hz, 1H); 2,37(m, 1H); 3,73(dxdxd, $J_1$=12, $J_2$=12, $J_3$=4Hz, 1H); 5,40(d, J=4Hz, 1H); 6,07(d, j=4Hz, 1H) δ ppm. |

| | |
|---|---|
| RMN($^{13}C$, 360MHz, $CDCl_3$) : | 22,0(q); 25,0(t); 31,4(d); 33,8(t); 38,6(t); 48,7(d); 82,6(d); 116,9(t); 139,8(s); 170,7(s) δ ppm. |
| SM : | 166(4, $M^+$), 138(40), 109(36), 94(100), 81(73), 67(65), 55(47), 41(25). |
| Odeur : | coumarinée, lactonique, tonka, jonquille, très puissante. |

(3aS,6R,7aS)-perhydro-6-méthyl-3-méthylène-2-benzo[b]furanone

| | |
|---|---|
| RMN($^1H$, 360MHz, $CDCl_3$) : | 0,94(d, J=6Hz, 3H); 1,00(m, 1H); 1,25-1,40(m, 2H); 1,45-1,70(m, 2H); 1,85(m, 1H); 2,20(dxm, J=14Hz, 1H); 2,84(m, 1H); 2,52(m, 1H); 5,54(d, J=1Hz, 1H); 6,10(d, J=1Hz, 1H) δ ppm |
| RMN($^{13}C$, 360MHz, $CDCl_3$) : | 21,7(q); 25,6(d); 28,3(t); 31,2(t); 35,8(t); 39,5(d); 77,2(d); 119,5(t); 142,4(s); 170,9(s) δ ppm. |
| Odeur : | coumarinée, lactonique, tonka. |

H. Préparation de (-)-(3aS,6R,7aS)-perhydro-3,3,6-triméthyl-2-benzo[b] furanone

On a placé 2,54 g (35,8 mmole) de pyrrolidine dans un ballon à -40°, puis ajouté 14,3 ml de butyllithium 2,5 M dans l'hexane. On a laissé remonter la température à 0° puis de nouveau refroidi à -40° et introduit 5 g (29,8 mmole) de perhydro-3,6-diméthyl-2-benzo[b]furanone (mélange de 4 diastéréomères). On a laissé remonter la température à 0° et ajouté 12,8 g (90,3 mmole) d'iodure de méthyle avant de revenir à température ambiante. Après 1 h, le mélange de la réaction a été hydrolysé et extrait à l'éther. La phase organique a été lavée à l'eau, puis séchée sur $MgSO_4$ et concentrée. Le produit brut a été chromatographié sur silice (éluant: $Et_2O$/pentane = 3/7) pour fournir ainsi la lactone désirée (rend. ~10%).
$[\alpha]_D^{20}$ = -35,5° ; c = 0,65% dans le $CHCl_3$

| | |
|---|---|
| RMN($^1H$, 360MHz, $CDCl_3$) : | 0,8-0,95(m, 1H); 0,92(d, J=7Hz, 3H); 1,16(s, 3H); 1,29(s, 3H); 1,1-1,25(m, 2H); 1,53 (m, 1H); 1,62-1,75(m, 2H); 1,88(m, 1H); 2,24(m, 1H); 4,69(m, 1H) δ ppm. |
| RMN($^{13}C$, 360MHz, $CDCl_3$) : | 19,16(q); 21,93(q); 23,27(q); 24,89(t); 26,27(d); 32,32(t); 36,41(t); 44,80(d); 45,66(s); 76,21(d); 182,11(s) δ ppm. |
| SM : | 182(12), 132(2), 123(24), 95(100), 81(87), 67(38), 19(55), 41(7). |
| Odeur : | lactonique, fruitée, coumarinée. |

Les énantiomères suivants des furanones décrites dans les alinéas A à F présentaient les propriétés citées ci-après :

(3S,3aR,6S,7aS)-perhydro-3,6-diméthyl-2-benzol[b]furanone (pure à 87%)

Odeur : butyrique, rance, coumarinée, lactonique, soufrée, puissante.

(+)-(3R,3aR,6S,7aR)-perhydro-3,6-diméthyl-2-benzo[b]furanone $[\alpha]_D^{20}$ = +69,9°

Odeur : coumarinée, foin, terreuse, lactonique.

(+)-(3S,3aR,6S,7aR)-perhydro-3,6-diméthyl-2-benzo[b]furanone $[\alpha]_D^{20}$ = +45,8°

Odeur : coumarinée, noix, huile de noix.

Les (3S,3aS,6S,7aR)-perhydro-3,6-diméthyl-2-benzo[b]furanone, (3S,3aS,6S,7aS)-perhydro-3,6-diméthyl-2-benzo[b]furanone, (3R,3aR,6S,7aS)-perhydro-3,6-diméthyl-2-benzo[b]furanone et (3R,3aS,6S,7aR)-perhydro-3,6-diméthyl-2-benzo[b]furanone ont également été préparées.

Les applications en parfumerie selon l'invention seront maintenant décrites plus en détail à l'aide des exemples suivants.

Exemple 1

Préparation d'une composition parfumante

On a préparé une composition parfumante de base, destinée à un parfum de type masculin, oriental, en mélangeant

les ingrédients suivants:

| Ingrédients | Parties en poids |
|---|---|
| Acétate de linalyle | 210 |
| Ambrox® DL[1)] à 10%* | 350 |
| Anthranilate de méthyle dist. | 25 |
| 4-(4-Hydroxyphényl)-2-butanone à 10%* | 15 |
| Essence de bergamote synth. | 135 |
| Essence de cannelle de Ceylan à 10%* | 70 |
| Eugénol à 10%* | 40 |
| Essence de citron | 110 |
| Héliotropine ord. | 120 |
| Hydroxycitronellal | 35 |
| Indol purif. à 10%* | 15 |
| Polywood® [2)] | 190 |
| Dihydromyrcénol [3)] | 180 |
| Linalol | 50 |
| Lyral® [4)] | 400 |
| Essence de mandarine synth. | 30 |
| Iralia® [5)] | 90 |
| Méthylnaphtylcétone cryst. | 20 |
| Jasmonate de méthyle | 300 |
| Essence de patchouli | 120 |
| Sandalore® [6)] | 50 |
| Tonalid® [7)] | 500 |
| Exaltolide® [8)] | 200 |
| Vanilline | 95 |
| Vertofix coeur [9)] | 250 |
| TOTAL | 3600 |

* dans le dipropylèneglycol

1) tétraméthyl perhydronaphtofurane, origine: Firmenich SA, Genève, Suisse

2) acétate de perhydro-5,5,8a-triméthyl-2-naphtyle ; origine : Firmenich SA, Genève, Suisse

3) origine : International Flavors and Fragrances Inc., U.S.A.

4) 4-(4-hydroxy-4-méthylpentyl)-3-cyclohexéne-1-carboxaldéhyde ; origine : International Flavors and Fragrances Inc., U.S.A.

5) méthylionone; origine: Firmenich SA, Genève, Suisse

6) 5-(2,2,3-triméthylcyclopent-3-ényl)-3-méthylpentan-2-ol ; origine : L. Givaudan, Vernier, Suisse

7) 7-acétyl-1,1,3,4,4,6-hexaméthyltétraline ; origine : PFW, Hollande

8) pentadécanolide; origine: Firmenich SA, Genève, Suisse

9) origine: International Flavors and Fragrances Inc., U.S.A.

A cette composition de base de type balsamique-floral, boisé, musqué, on a ajouté 400 parties en poids de coumarine pour obtenir une composition A, respectivement 400 parties en poids de perhydro-3,6-diméthyl-2-benzo[b] furanone pour obtenir une composition B nouvelle.

Ces deux compositions A et B ont été comparées à l'aveugle par un panel d'experts parfumeurs. De l'avis de ces derniers la composition B nouvelle développait une odeur beaucoup plus balsamique que celle de la composition A, odeur qui rappelait celle des fèves de tonka. Par ailleurs, le caractère floral de la composition B semblait complètement étouffé à cette concentration de furanone.

Lorsqu'on a réduit de moitié la quantité de perhydro-3,6-diméthyl-2-benzo[b]furanone ajoutée à la composition de base, on a obtenu une composition nouvelle dont l'odeur se rapprochait beaucoup plus de celle de la composition A, avec toutefois un caractère moins vanillé.

Lorsqu'on a ajouté à la composition de base 100 parties en poids de perhydro-3,6-diméthyl-2-benzo[b]furanone on a obtenu un effet olfactif encore plus semblable à celui qu'on obtenait avec 400 parties en poids de coumarine, c'est-à-dire, on a obtenu une composition nouvelle dont l'odeur était très proche de celle de la composition A, quoique globalement plus boisée et moins vanillée.

On a également comparé la performance de cette furanone avec celle de son homologue insaturé, la 5,6,7,7a-tétrahydro-7a-méthoxy-3,6-diméthyl-2(4H)-benzo[b]furanone ou menthalactone.
Lorsqu'on a ajouté à deux échantillons distincts de la composition de base susmentionnée 200 parties en poids de la furanone selon la présente invention et 200 parties en poids de menthalactone, on a obtenu deux compositions, respectivement C et D, possédant des odeurs tout à fait différentes. Alors que l'odeur de la composition nouvelle C, contenant la perhydro-3,6-diméthyl-2-benzo[b]furanone, avait comme caractère dominant la note orientale, vanillée-coumarinée, celle de la composition D, qui contenait la menthalactone, était vaguement poudrée-vanillée, avec un caractère de type "alimentaire", la note vanillée étouffant toute la fragrance de la composition D. Contrairement à cette dernière, la composition C avait une odeur clairement parfumistique, riche, avec beaucoup plus de volume et une note élégante de type oriental-coumariné.
D'autre part, la composition D semblait beaucoup moins "concentrée" que la composition C.

Exemple 2

Test de substantivité sur du linge

On a préparé des échantillons d'une base adoucissante parfumée en ajoutant à des échantillons distincts d'un adoucissant textile standard non parfumé respectivement de la perhydro-3,6-diméthyl-2-benzo[b]furanone, de la coumarine, de la 8-oxatricyclo[5.3.1.$0^{2,6}$]undécan-9-one (origine : Firmenich SA; voir brevet US 3,981,892), de la cis-10,10-diméthyl-tricyclo [7.1.1.$0^{2,7}$]undéc-2-én-4-one (origine: Firmenich SA; voir brevet US 4,226,745), de la 1-oxaspiro[4.5]décan-2-one (origine: Firmenich SA; voir brevet US 5,057,239), et de la menthalactone dans les quantités indiquées dans le Tableau (parties en poids) ci-après.

Tableau

| Ingrédients | Echantillon | | | | | |
|---|---|---|---|---|---|---|
| | 1 | 2 | 3 | 4 | 5 | 6 |
| adoucissant non-parfumé | 99,9 | 99,9 | 99,9 | 99,9 | 99,9 | 99,9 |
| perhydro-3,6-diméthyl-2-benzo[b]furanone | 0,1 | - | - | - | - | - |
| coumarine | - | 0,1 | - | - | - | - |
| 8-oxatricyclo[5.3.1.$0^{2,6}$]undécan-9-one | - | - | 0,1 | - | - | - |
| cis-10,10-diméthyltricyclo[7.1.1.$0^{2,7}$]undéc-2-én-4-one | - | - | - | 0,1 | - | - |
| 1-oxaspiro[4.5]décan-2-one | - | - | - | - | 0,1 | - |
| menthalactone | - | - | - | - | - | 0,1 |

Dans six machines à laver le linge, on a traité séparément six lots de textiles standard, contenant des textiles en coton, en fibre acrylique et en nylon, avec respectivement les échantillons 1 à 6 préparés ci-dessus. Les six lots de textiles ainsi traités ont ensuite été évalués à l'aveugle par un panel d'experts parfumeurs, aussi bien à l'état humide qu'après leur séchage. Les résultats de ces essais comparatifs ont montré que, de l'avis des parfumeurs, le lot de textiles traité avec l'échantillon 1, contenant la perhydro-3,6-diméthyl-2-benzo[b]furanone, exhalait une odeur jugée cinq à dix fois plus forte que l'odeur du lot de textiles traité avec l'échantillon 2, contenant de la coumarine, ceci aussi bien sur le linge humide que sur le linge sec. L'odeur du linge traité avec l'échantillon 1 était aussi beaucoup plus durable après le séchage.
Les textiles traités avec l'échantillon 3 exhalaient une odeur jugée moins puissante et moins substantive que celle des textiles traités avec l'échantillon 2, alors que l'odeur du lot traité avec l'échantillon 4 était plus puissante et tenace que celle de ces derniers, et que l'odeur exhalée par les textiles traités avec l'échantillon 5 était comparable, en intensité et durabilité, à l'odeur des textiles traités avec l'échantillon 2. De l'avis unanime des parfumeurs, tous ces trois lots de textiles traités avec les échantillons 3, 4 et 5 possédaient des odeurs qui étaient nettement inférieures, en puissance et substantivité, à l'odeur du lot traité avec l'échantillon 1, ceci aussi bien à la sortie de la machine à laver qu'après le séchage des textiles.
Par ailleurs, les textiles traités avec l'échantillon 6 développaient une odeur beaucoup plus faible à l'état humide que ceux traités avec l'échantillon 1, odeur qui disparaissait très vite sur les textiles secs. Ce lot de textiles était, en fait, le moins substantif de tous les lots susmentionnés.

**Revendications**

1. Utilisation d'une furanone de formule

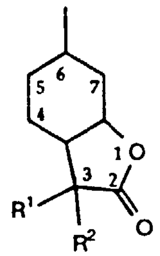

(I)

dans laquelle les symboles $R^1$ et $R^2$, pris séparément, sont identiques et représentent chacun un radical méthyle, ou sont différents et représentent chacun un atome d'hydrogène ou un radical méthyle, ou, pris ensemble, représentent un radical méthylène, à titre d'ingrédient parfumant pour la préparation de compositions parfumantes ou d'articles parfumés.

2. Utilisation selon la revendication 1, caractérisée en ce que ladite furanone se présente sous forme de l'un de ses isomères optiquement actifs.

3. Utilisation selon la revendication 2, de la (+)-(3aS,6R,7aR)-perhydro-6-méthyl-3-méthylène-2-benzo[b]furanone ou de la (3aS,7aR)-perhydro-6-méthyl-2-benzo[b]furanone.

4. Utilisation selon la revendication 2, de la perhydro-3,6-diméthyl-2-benzo[b]furanone, sous forme de l'un des composées suivants:

   a) (+)-(3R,3aS,6R,7aR)-perhydro-3,6-diméthyl-2-benzo[b]furanone
   b) (+)-(3S,3aS,6R,7aR)-perhydro-3,6-diméthyl-2-benzo[b]furanone
   c) (-)-(3S,3aR,6R,7aS)-perhydro-3,6-diméthyl-2-benzo[b]furanone.

5. Utilisation selon la revendication 1, de la perhydro-3,6-diméthyl-2-benzo[b]furanone, sous forme d'un mélange de deux ou plusieurs de ses isomères optiquement actifs.

6. Utilisation selon la revendication 5, caractérisée en ce que le poids combiné des (+)-(3R,3aS,6R,7aR)-perhydro-3,6-diméthyl-2-benzo[b]furanone et (+)-(3S,3aS,6R,7aR)-perhydro-3,6-diméthyl-2-benzo[b]furanone dans ledit mélange est de 50% ou plus.

7. Utilisation selon la revendication 6, caractérisée en ce que ledit mélange contient environ 50% en poids de (+)-(3S,3aS,6R,7aR)-perhydro-3,6-diméthyl-2-benzo[b]furanone, 30% en poids de (+)-(3R,3aS,6R,7aR)-perhydro-3,6-diméthyl-2-benzo[b]furanone, 11% en poids de (-)-(3S,3aS,6R,7aS)-perhydro-3,6-diméthyl-2-benzo[b]furanone et 7% en poids de (-)-(3R,3aS,6R,7aS)-perhydro-3,6-diméthyl-2-benzo[b]furanone, par rapport au poids du mélange.

8. Utilisation selon la revendication 5, caractérisée en ce que ledit mélange contient 50% ou plus de l'un des isomères suivants :

   a) (+)-(3R,3aS,6R,7aR)-perhydro-3,6-diméthyl-2-benzo[b]furanone
   b) (+)-(3S,3aS,6R,7aR)-perhydro-3,6-diméthyl-2-benzo[b]furanone.

9. Composition parfumante ou article parfumé résultant de l'utilisation selon l'une des revendications 1 à 8.

10. Article parfumé selon la revendication 9, sous forme d'un parfum ou d'une eau de toillette, d'un savon, d'un gel de douche ou bain, d'un shampoing ou autre produit d'hygiène capillaire, d'une préparation cosmétique, d'un désodorisant corporel ou d'air ambiant, d'un détergent ou adoucissant textile, ou d'un produit d'entretien.

**11.** Composition contenant 50% ou plus de l'un des composés suivants :

a) (+)-(3S,3aS,6R,7aR)-perhydro-3,6-diméthyl-2-benzo[b]furanone
b) (+)-(3R,3aS,6R,7aR)-perhydro-3,6-diméthyl-2-benzo[b]furanone.

**12.** Composition caractérisée en ce qu'au moins 50% de son poids est constitué par un mélange de (+)-(3S,3aS,6R,7aR)-perhydro-3,6-diméthyl-2-benzo[b]furanone et (+)-(3R,3aS,6R,7aR)-perhydro-3,6-diméthyl-2-benzo[b]furanone.

**13.** Composition selon la revendication 12, contenant environ 50% en poids de (+)-(3S,3aS,6R,7aR)-perhydro-3,6-diméthyl-2-benzo[b]furanone, 30% en poids de (+)-(3R,3aS,6R,7aR)-perhydro-3,6-diméthyl-2-benzo[b]furanone, 11% en poids de (-)-(3S,3aS,6R,7aS)-perhydro-3,6-diméthyl-2-benzo[b]furanone et 7% en poids de (-)-(3R,3aS,6R,7aS)-perhydro-3,6-diméthyl-2-benzo[b]furanone, par rapport au poids de composition.

**14.** Composé choisi parmi les suivants:

a. (-)-(3S,3aS,6R,7aS)-perhydro-3,6-diméthyl-2-benzo[b]furanone
b. (-)-(3R,3aS,6R,7aS)-perhydro-3,6-diméthyl-2-benzo[b]furanone
c. (-)-(3R,3aR,6R,7aS)-perhydro-3,6-diméthyl-2-benzo[b]furanone
d. (-)-(3S,3aR,6R,7aS)-perhydro-3,6-diméthyl-2-benzo[b]furanone
e. (3S,3aR,6S,7aS)-perhydro-3,6-diméthyl-2-benzo[b]furanone
f. (+)-(3R,3aR,65,7aR)-perhydro-3,6-diméthyl-2-benzo[b]furanone
g. (+)-(3S,3aR,6S,7aR)-perhydro-3,6-diméthyl-2-benzo[b]furanone
h. (3S,3aS,6S,7aR)-perhydro-3,6-diméthyl-2-benzo[b]furanone
i. (3S,3aS,6S,7aS)-perhydro-3,6-diméthyl-2-benzo[b]furanone
j. (3R,3aS,6S,7aR)-perhydro-3,6-diméthyl-2-benzo[b]furanone
k. (-)(3aS,6R,7aS)-perhydro-3,3,6-triméthyl-2-benzo[b]furanone.

**15.** Utilisation d'une furanone telle que définie dans l'une des revendications 1 à 4 pour la reconstitution de l'odeur caractéristique de la coumarine dans des compositions parfumantes ou articles parfumés.

**16.** Utilisation selon la revendication 15, caractérisée en ce que ladite furanone se présente sous forme d'un mélange tel que défini dans l'une des revendications 5 à 8.

**Patentansprüche**

**1.** Verwendung eines Furanons der Formel

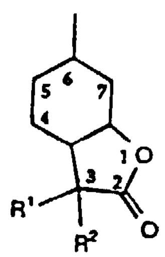

(I)

worin die Symbole $R^1$ und $R^2$ als getrennte Gruppen identisch sind und jedes für einen Methylrest steht oder verschieden sind und jedes für ein Wasserstoffatom oder einen Methylrest steht oder zusammen einen Methylenrest bedeuten als Riechstoff für die Herstellung von Riechstoffzusammensetzungen oder parfümierten Artikeln.

**2.** Verwendung gemäss Patentanspruch 1, dadurch gekennzeichnet, dass sich das genannte Furanon in Form eines seiner optisch aktiven Isomere befindet.

**3.** Verwendung gemäss Patentanspruch 2 des (+)-(3aS,6R,7aR)-Perhydro-6-methyl-3-methylen-2-benzo[b]furanons oder des (3aS,7aR)-Perhydro-6-methyl-2-benzo[b]furanons.

**4.** Verwendung gemäss Patentanspruch 2 des Perhydro-3,6-dimethyl-2-benzo[b]furanons in Form einer der folgenden Verbindungen:

a) (+)-(3R,3aS,6R,7aR)-Perhydro-3,6-dimethyl-2-benzo[b]furanon
b) (+)-(3S,3aS,6R,7aR)-Perhydro-3,6-dimethyl-2-benzo[b]furanon
c) (-)-(3S,3aR,6R,7aS)-Perhydro-3,6-dimethyl-2-benzo[b]furanon.

**5.** Verwendung gemäss Patentanspruch 1 des Perhydro-3,6-dimethyl-2-benzo[b]furanons in Form einer Mischung von zwei oder mehreren seiner optisch aktiven Isomeren.

**6.** Verwendung gemäss Patentanspruch 5, dadurch gekennzeichnet, dass das Gesamtgewicht von (+)-(3R,3aS,6R,7aR)-Perhydro-3,6-dimethyl-2-benzo[b]furanon und (+)-(35,3aS,6R,7aR)-Perhydro-3,6-dimethyl-2-benzo[b]furanon in dieser Mischung 50% oder mehr beträgt.

**7.** Verwendung gemäss Patentanspruch 6, dadurch gekennzeichnet, dass diese Mischung ungefähr 50 Gew.% (+)-(3S,3aS,6R,7aR)-Perhydro-3,6-dimethyl-2-benzo[b]furanon, 30 Gew.% (+)-(3R,3aS,6R,7aR)-Perhydro-3,6-dimethyl-2-benzo[b]furanon, 11 Gew.% (-)-(3S,3aS,6R,7aS)-Perhydro-3,6-dimethyl-2-benzo[b]furanon und 7 Gew.% (-)-(3R,3aS,6R,7aS)-Perhydro-3,6-dimethyl-2-benzo[b]furanon, bezogen auf das Gewicht der Mischung, enthält.

**8.** Verwendung gemäss Patentanspruch 5, dadurch gekennzeichnet, dass die genannte Mischung 50 % oder mehr eines der folgenden Isomere enthält:

a) (+)-(3R,3aS,6R,7aR)-Perhydro-3,6-dimethyl-2-benzo[b]furanon
b) (+)-(3S,3aS,6R,7aR)-Perhydro-3,6-dimethyl-2-benzo[b]furanon.

**9.** Riechstoffzusammensetzung oder parfümierter Artikel, welche sich aus der Verwendung gemäss einem der Patentansprüche 1 bis 8 ergeben.

**10.** Parfümierter Artikel gemäss Patentanspruch 9 in Form eines Parfüms und eines Eau de Toilette, einer Seife, eines Dusche- oder Badegels, eines Shampoos oder eines anderen Haarpflegeproduktes, einer kosmetischen Zubereitung, eines Körperdesodorans oder eines Raumluftverbesserers, eines Detergens oder Textilweichmachers oder eines Universalhaushaltreinigers.

**11.** Zusammensetzung, welche 50% oder mehr einer der folgenden Verbindungen enthält:

a) (+)-(3S,3aS,6R,7aR)-Perhydro-3,6-dimethyl-2-benzo[b]furanon
b) (+)-(3R,3aS,6R,7aR)-Perhydro-3,6-dimethyl-2-benzo[b]furanon.

**12.** Zusammensetzung, dadurch gekennzeichnet, dass mindestens 50% ihres Gewichtes aus einer Mischung von (+)-(3S,3aS,6R,7aR)-Perhydro-3,6-dimethyl-2-benzo[b]furanon und (+)-(3R,3aS,6R,7aR)-Perhydro-3,6-dimethyl-2-benzo[b]furanon besteht.

**13.** Zusammensetzung gemäss Patentanspruch 12, welche ungefähr 50 Gew.% (+)-(3S,3aS,6R,7aR)-Perhydro-3,6-dimethyl-2-benzo[b]-furanon, 30 Gew.% (+)-(3R,3aS,6R,7aR)-Perhydro-3,6-dimethyl-2-benzo[b]furanon, 11 Gew.% (-)-(3S,3aS,6R,7aS)-Perhydro-3,6-dimethyl-2-benzo[b]furanon und 7 Gew.% (-)-(3R,3aS, 6R,7aS)-Perhydro-3,6-dimethyl-2-benzo[b]furanon, bezogen auf das Gewicht der Zusammensetzung, enthält.

**14.** Verbindung, ausgewählt aus den folgenden:

a. (-)-(3S,3aS,6R,7aS)-Perhydro-3,6-dimethyl-2-benzo[b]furanon
b. (-)-(3R,3aS,6R,7aS)-Perhydro-3,6-dimethyl-2-benzo[b]furanon
c. (-)-(3R,3aR,6R,7aS)-Perhydro-3,6-dimethyl-2-benzo[b]furanon
d. (-)-(3S,3aR,6R,7aS)-Perhydro-3,6-dimethyl-2-benzo[b]furanon
e. (3S,3aR,6S,7aS)-Perhydro-3,6-dimethyl-2-benzo[b]furanon
f. (+)-(3R,3aR,6S,7aR)-Perhydro-3,6-dimethyl-2-benzo[b]furanon
g. (+)-(3S,3aR,6S,7aR)-Perhydro-3,6-dimethyl-2-benzo[b]furanon
h. (3S,3aS,6S,7aR)-Perhydro-3,6-dimethyl-2-benzo[b]furanon

i. (3S,3aS,6S,7aS)-Perhydro-3,6-dimethyl-2-benzo[b]furanon
j. (3R,3a5, 65,7aR)-Perhydro-3,6-dimethyl-2-benzo[b]furanon
k. (-)-(3aS,6R,7aS)-Perhydro-3,3,6-trimethyl-2-benzo[b]furanon.

**15.** Verwendung eines Furanons, das in einem der Patentansprüche 1 bis 4 definiert ist, zur Wiederherstellung des charakteristischen Duftes von Cumarin in Riechstoffzusammensetzungen oder parfümierten Artikeln.

**16.** Verwendung gemäss Patentanspruch 15, dadurch gekennzeichnet, dass sich das genannte Furanon in Form einer Mischung befindet, die in einem der Patentansprüche 5 bis 8 definiert wird.

## Claims

**1.** Use of a furanone of formula

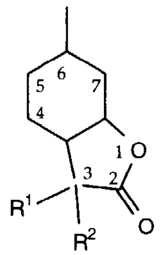

(I)

wherein symbols $R^1$ and $R^2$, taken separately, are identical and represent each a methyl radical, or are different and represent each a hydrogen atom or a methyl radical, or, taken together, represent a methylene radical, as a perfuming ingredient for the preparation of perfuming compositions or perfumed articles.

**2.** Use according to claim 1, characterized in that said furanone is in the form of one of its optically active isomers.

**3.** Use according to claim 2, of (+)-(3aS,6R,7aR)-perhydro-6-methyl-3-methylene-2-benzo[b]furanone or of (3aS, 7aR)-perhydro-6-methyl-2-benzo[b]furanone.

**4.** Use according to claim 2, of perhydro-3,6-dimethyl-2-benzo[b] furanone, in the form of one of the following compounds:

a) (+)-(3R,3aS,6R,7aR)-perhydro-3,6-dimethyl-2-benzo[b]furanone
b) (+)-(3S,3aS,6R,7aR)-perhydro-3,6-dimethyl-2-benzo[b]furanone
c) (-)-(3S,3aR,6R,7aS)-perhydro-3,6-dimethyl-2-benzo[b]furanone.

**5.** Use according to claim 1, of perhydro-3,6-dimethyl-2-benzo[b] furanone, in the form of a mixture of two or several of its optically active isomers.

**6.** Use according to claim 5, characterized in that the combined weight of (+)-(3R,3aS,6R,7aR)-perhydro-3,6-dimethyl-2-benzo[b]furanone and (+)-(3S,3aS,6R,7aR)-perhydro-3,6-dimethyl-2-benzo[b]furanone in said mixture is 50% or more.

**7.** Use according to claim 6, characterized in that said mixture contains about 50% by weight of (+)-(3S,3aS,6R,7aR)-perhydro-3,6-dimethyl-2-benzo[b]furanone, 30% by weight of (+)-(3R,3aS,6R,7aR)-perhydro-3,6-dimethyl-2-benzo[b]furanone, 11% by weight of (-)-(3S,3aS,6R,7aS)-perhydro-3,6-dimethyl-2-benzo[b]furanone and 7% by weight of (-)-(3R,3aS,6R,7aS)-perhydro-3,6-dimethyl-2-benzo[b]furanone, relative to the weight of the mixture.

**8.** Use according to claim 5, characterized in that said mixture contains 50% or more of one of the following isomers:

a) (+)-(3R,3aS,6R,7aR)-perhydro-3,6-dimethyl-2-benzo[b]furanone
b) (+)-(3S,3aS,6R,7aR)-perhydro-3,6-dimethyl-2-benzo[b]furanone.

**9.** Perfuming composition or perfumed article resulting from the use according to any one of claims 1 to 8.

**10.** Perfumed article according to claim 9, in the form of a perfume or a cologne, a soap, a bath or shower gel, a shampoo or other hair-care product, a cosmetic preparation, a body or air deodorant, a detergent or a fabric softener, or a household product.

**11.** Composition containing 50% or more of one of the following compounds :

a) (+)-(3S,3aS,6R,7aR)-perhydro-3,6-dimethyl-2-benzo[b]furanone
b) (+)-(3R,3aS,6R,7aR)-perhydro-3,6-dimethyl-2-benzo[b]furanone.

**12.** Composition characterized in that at least 50% of its weight is formed of a mixture of (+)-(3S,3aS,6R,7aR)-perhydro-3,6-dimethyl-2-benzo[b] furanone and (+)-(3R,3aS,6R,7aR)-perhydro-3,6-dimethyl-2-benzo[b]furanone.

**13.** Composition according to claim 12, containing around 50% by weight of (+)-(3S,3aS,6R,7aR)-perhydro-3,6-dimethyl- [b]furanone, 30% by weight of (+)-(3R,3aS,6R,7aR)-perhydro-3,6-dimethyl-2-benzo[b]furanone, 11% by weight of (-)-(3S,3aS,6R,7aS)-perhydro-3,6-dimethyl-2-benzo[b] furanone and 7% by weight of (-)-(3R,3aS,6R,7aS)-perhydro-3,6-dimethyl-2-benzo[b]furanone, relative to the weight of the composition.

**14.** Compound chosen amongst the following:

a. (-)-(3S,3aS,6R,7aS)-perhydro-3,6-dimethyl-2-benzo[b]furanone
b. (-)-(3R,3aS,6R,7aS)-perhydro-3,6-dimethyl-2-benzo[b]furanone
c. (-)-(3R,3aR,6R,7aS)-perhydro-3,6-dimethyl-2-benzo[b]furanone
d. (-)-(3S,3aR,6R,7aS)-perhydro-3,6-dimethyl-2-benzo[b]furanone
e. (3S,3aR,6S,7aS)-perhydro-3,6-dimethyl-2-benzo[b]furanone
f. (+)-(3R,3aR,6S,7aR)-perhydro-3,6-dimethyl-2-benzo[b]furanone
g. (+)-(3S,3aR,6S,7aR)-perhydro-3,6-dimethyl-2-benzo[b]furanone
h. (3S,3aS,6S,7aR)-perhydro-3,6-dimethyl-2-benzo[b]furanone
i. (3S,3aS,6S,7aS)-perhydro-3,6-dimethyl-2-benzo[b]furanone
j. (3R,3aS,6S,7aR)-perhydro-3,6-dimethyl-2-benzo[b]furanone
k. (-)-(3aS,6R,7aS)-perhydro-3,3,6-triméthyl-2-benzo[b]furanone.

**15.** Use of a furanone such as defined in any one of claims 1 to 4 for reconstituting the odor characteristic of coumarine in perfuming compositions and perfumed articles.

**16.** Use according to claim 15, characterized in that said furanone is in the form of a mixture such as defined in any one of claims 5 to 8.